# EUROPÄISCHE PATENTSCHRIFT

(11) **EP 1 444 972 B1**
(45) Veröffentlichungstag und Bekanntmachung des Hinweises auf die Patenterteilung: **29.03.2006**
(21) Anmeldenummer: 04000899.7
(22) Anmeldetag: 16.01.2004
(51) Int. Cl.: A61K 6/00, A61K 6/02

(54) **Härtbare Dentalmassen**
Hardenable dental material
Matériau dentaire durcissable

(30) Priorität: 05.02.2003 DE 10304758
(43) Veröffentlichungstag der Anmeldung: 11.08.2004
(73) Patentinhaber: Heraeus Kulzer GmbH, 63450 Hanau (DE)
(72) Erfinder: Finger, Werner, Professor Dr., 41469 Neuss (DE)
(74) Vertreter: Kühn, Hans-Christian

(56) Entgegenhaltungen:
- EP-A- 0 591 716
- EP-A- 1 269 967
- DE-A- 10 124 028
- GB-A- 2 075 504
- US-A- 5 425 641

## Beschreibung

Die Erfindung betrifft härtbare Dentalmassen, die feinteiligen Anionenaustauscher enthalten, ein Verfahren zu Ihrer Herstellung und ihre Verwendung.

Härtbare Dentalmassen, ähnlich wie sie in dieser Erfindung beschrieben werden, finden in breitem Maße Verwendung in der Zahnmedizin z.B. als Haftvermittler, adhäsive Zahnlacke, Versiegler oder Befestigungsmaterialien oder Dentalfüllungsmaterial. Eine Verwendung in der Zahntechnik, z.B. bei der Anbindung polymerer oder nichtpolymerer Materialien an metallische Gerüstteile ist ebenfalls möglich.

Übliche härtbare Dentalmassen enthalten als einen wesentlichen Bestandteil (Meth)acrylatmonomere, die durch radikalisch initiierte Polymerisation in vemetzte Polymere umgewandelt werden und auf diese Weise die Verfestigung bzw. Aushärtung der Dentalmasse bewirken. Für die Initiierung der Polymerisation kommen dabei in erster Linie Photoinitiatoren, durch Wärme aktivierbare Initiatoren und Redoxsysteme in Frage.

Photoinitiatoren ermöglichen die Formulierung von Einkomponentensystemen, die durch Besirahiung mit intensiven Lichtquellen ausgehärtet werden können. Eine derartige photohärtbare Zubereitung zur Behandlung der Zahnhartsubstanz wird beispielsweise in der US 5,849,270 eingehend beschrieben.

Für Einsatzgebiete, bei denen die Aushärtung mittels Bestrahlung ungünstig oder unmöglich ist, wie z. B. bei der Befestigung von Kronen aus lichtundurchlässigem Material, kann die Redoxpolymerisation angewendet werden. In diesem Fall werden zwei Komponenten, von denen die eine einen Initiator, z.B. Dibenzoylperoxid, und die andere einen Coaktivator, z.B. ein Amin wie N,N-Dimethyl-p-Touidin, enthält, zur Einleitung der Aushärtung intensiv gemischt.

Um eine gute Haftung der gehärteten Dentalmasse an der Zahnsubstanz zu erreichen, kann die Zahnsubstanz vor dem Auftragen der Dentalmasse mit einer Flüssigkeit konditioniert werden. Diese Konditionierflüssigkeit enthält im Allgemeinen Säuren mit einem pKs-Wert kleiner als 5 und besitzt einen pH-Wert von 0,1 bis 3,5. Geeignete Säuren sind in diesem Zusammenhang beispielsweise Phosphorsäure, Salpetersäure, Brenztraubensäure, Zitronensäure, Oxalsäure, Ethylendiamintetraessigsäure, Weinsäure und Essigsäure.

Anstelle von Konditionierflüssigkeiten werden in zunehmenden Maße selbstätzende Primer eingesetzt, bei denen mittels saurer Monomere gleichzeitig die Ätzung der Zahnhartsubstanz und die Anbindung an freigelegtes Dentinkollagen ermöglicht wird. Solche selbstätzenden Primer sind leicht zu verarbeiten, und sie zeigen eine hohe Haftkraft an der Zahnsubstanz. Selbsthärtende Primer enthalten neben sauren Komponenten wie z.B. Polyacrylsäure, Maleinsäure oder 4-(Meth)acryloxyethylphthalsäure, (Meth)acrylatmonomere und Initiatoren für die Aushärtung.

Bei bestimmten Anwendungen hat sich allerdings gezeigt, dass die Behandlung mit einer Konditionierflüssigkeit oder einem selbstätzenden Primer die Aushärtung von anschließend aufgebrachten Dentalmassen behindert (Sanares AME, Itthagarun A, King NM, Tay FR, Pashley DH. Adverse surface interactions between one-bottle light-cured adhesives and chemical-cured composites. *Dental Materials* 2001;17:542-556). Diese Behinderung äußert sich in einer verlangsamten oder unvollständigen Aushärtung der Dentalmasse. Besonders ausgeprägt ist diese Behinderung bei der oben erwähnten Redoxpolymerisation von Zweikomponenten Systemen.

Die Aufgabe der vorliegenden Erfindung ist daher die Bereitstellung härtbarer Dentalmassen, die auch auf mit sauren Komponenten behandelten Zahnflächen vollständig aushärten.

Es wurde gefunden, dass ein Zusatz von feinteiligem Anionenaustauscher in der OH-Form zur härtbaren Dentalmasse deren vollständige Aushärtung auf Zahnflächen gewährleistet, auch dann wenn die Zahnflächen zuvor mit einer sauren Konditionierflüssigkeit oder einem selbstätzenden Primer behandelt wurden.

Gegenstand der Erfindung sind somit härtbare Dentalmassen aus Zweikomponentensystemen aus Peroxid- und Aminkomponenten, wobei die Aminkomponenten
a) radikalisch polymerisierbare Monomere,
b) aromatisches Amin als Coaktivator,
c) übliche Zuschlagsstoffe, und zusätzlich
d) feinteiligen Anionenaustauscher in der OH Form enthalten.

Die Erfindung betrifft auch die Verwendung solcher Aminkomponenten härtbarer Dentalmassen zur Herstellung von Dentalfüllungsmaterialien, Haftvermittlern, adhäsiven Zahnlacken, Versieglern oder Befestigungsmaterialien.

Radikalisch polymerisierbare Monomere (a) sind in erster Linie die Ester der Acrylsäure und Methacrylsäure. Bevorzugt sind Ester aus (Meth)-acrylsäure und 1- bis 5-wertigen Alkoholen mit 2 bis 30 Kohlenstoffatomen. Gut geeignet sind auch sogenannte Epoxyacrylate, wie das Bis-GMA der Formel 1

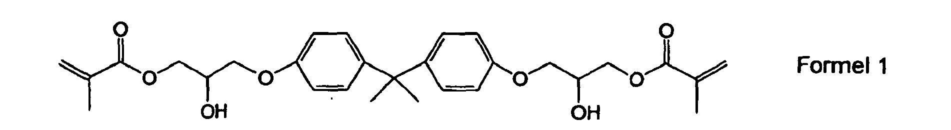

Des weiteren seinen Urethanacrylate genannt, die durch Umsetzung von Diisocyanaten und Hydroxyalkyl(meth)acrylaten hergestellt werden können. Beispiele für besonders gut geeignete Urethanacrylate sind:

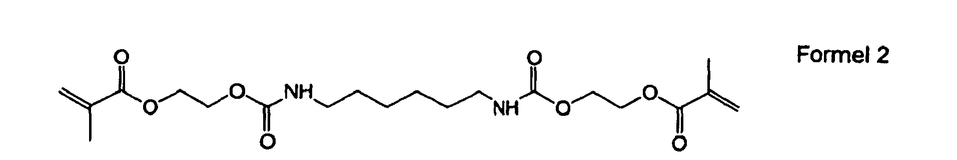

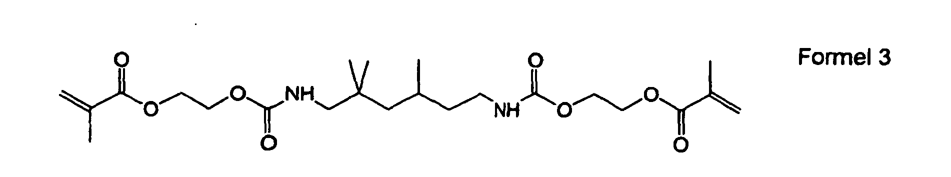

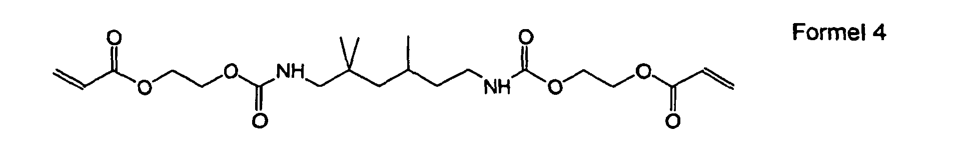

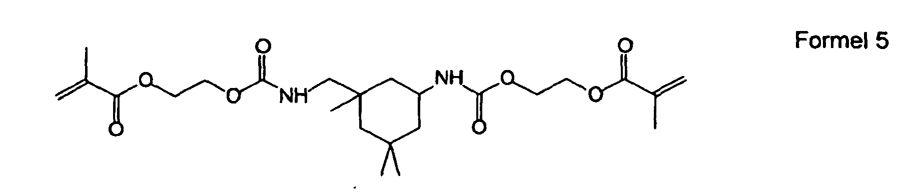

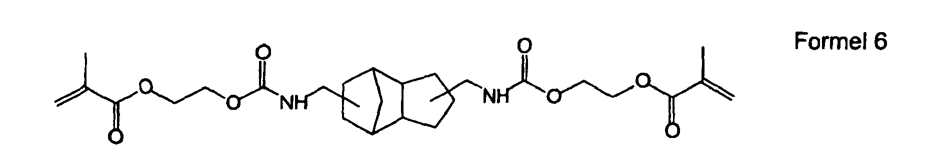

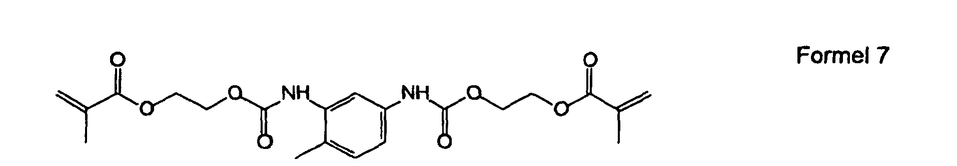

Die verwendeten Monomere bzw. Monomermischungen sollen eine Viskosität von 50 bis 5000 mPa.s, vorzugsweise von 100 bis 2000 mPa.s (jeweils bei 20 °C) besitzen. Höher viskose Monomere können mit niedrig viskosen Monomeren, sogenannten Reaktivverdünnern abgemischt werden. Geeignete Reaktivverdünner sind beispielsweise Triethylenglycol-dimethacrylat, Tetraethylenglycoldimethacrylat, Hexandioldimethacrylat, Neopentylglycol-dimethacrylat und Trimethylolpropantriacrylat. Trietylenglycoldimetacrylat ist bevorzugt. Im Allgemeinen wird durch Zusatz von 5 bis 40 Gew.-% Reaktivverdünner (bezogen auf die Summe der Monomere) eine geeignete Viskosität erreicht.
Es hat sich als besonders vorteilhaft herausgestellt, zumindest anteilsweise Ester der Acrylsäure und Methacrylsäure mit freien Hydroxylgruppen bei der Formulierung der erfindungsgemäßen Dentalmassen zu verwenden. Beispielhaft seien genannt Hydroxyethylmethacrylat, Hydroxyethylacrylat, Hydroxypropylmethacrylat. Glycerinmono-methacrylat, Glycerindimethacrylat. Hydroxyethylmethacrylat ist bevorzugt. Auch die Ester der Acrylsäure und Methacrylsäure mit freien Hydroxylgruppen wirken als Reaktivverdünner und setzen die Viskosität herab. Der Anteil an Ester der Acrylsäure und Methacrylsäure mit freien Hydroxylgruppen beträgt vorzugsweise 5 bis 30 Gew.-% bezogen auf die Summe der Monomere.

Radikalbildner (b) sind im vorliegendem Zusammenhang Stoffe, die unter Einwirkung von Licht, Wärme oder Zusatz eines Coinitiators freie Radikale liefern, die zur Auslösung einer Polymerisation geeignet sind.

Durch Licht aktivierbare Radikalbildner, die sogenannten Fotopolymerisationsinitiatoren, sind aus der Literatur bekannt. Es handelt sich um Mono- oder Dicarbonylverbindungen wie Benzoin und dessen Derivate, insbesondere Benzoylmethylether, Benzil und Benzilderivate und α-Diketoderivate insbesondere des Norbornans und substituierter Norbomane. Campherchinon ist bevorzugt. Die Fotopolymerisationsinitiatoren werden vorzugsweise mit Fotocoinitiatoren, wie z.B. Trihexylamin, N,N-Dimethylaminoethylmethacrylat, N,N,N',N',-Tetramethyletylendiamin und Dialkylbarbitursäure, kombiniert. Besonders geeignete Fotocoinitiatoren sind Dimethylaminobenzolsulfonsäureamide gemäss EP 73 995. Die Fotopolymerisationsinitiatoren werden in Mengen von 0,01 bis 2,5, vorzugsweise 0,1 bis 0,5 Gew.%, die Fotocoinitiatoren in Mengen von 0,02 bis 4, vorzugsweise 0,2 bis 1 Gew.-% jeweils bezogen auf die erfindungsgemäße Dentalmasse verwendet.

Radikalbildner die durch Wärme aktiviert werden können sind z.B. Azoverbindungen, wie z.B. 2,2'-Azobis(isobutyronitril) und 2,2'-Azobis(2-methylisobutyronitril) sowie Peroxy-verbindungen, wie Dibenzoylperoxid, Dilaurylperoxid, Bis(p-chlorbenzoylperoxid), Dicyclohexylperoxydicarbonat, tert.-Butylperoxy-2ethylhexanoat, 2,5-Bis(2-ethylhexanoyl-peroxy)-2,5-dimethylhexan, tert.-Butylperoxybenzoat und tert.-Amylperoxy-2-etylhexan. Es ist natürlich möglich und in vielen Fällen vorteilhaft, Mischungen von verschiedenen Radikalbildnern, beispielsweise von Radikalbildnern mit unterschiedlicher Zerfallstemperatur einzusetzen. Die Radikalbildner werden im allgemeinen in Mengen von 0.05 bis 2 Gew.-%, vorzugsweise 0,1 bis 0.8 Gew.% bezogen auf die Dentalmasse angewendet.

Zur Aushärtung bei Raumtemperatur kommt die erfindungsgemäße Dentalmasse als Zweikomponentensystem zur Anwendung, wobei die eine Komponente ein Peroxid als Radikalbildner und die andere ein aromatisches Amin als Coinitiator enthält. Die Zusammensetzung der Peroxid- und Aminkomponente kann bezüglich ihrer Hauptbestandteile (radikalisch polymerisierbare Monomere (b) und übliche Zusatzstoffe (c)) identisch sein. Im Hinblick auf eine gute Lagerstabilität der erfindungsgemäßen Dentalmasse wird jedoch der feinteilige Anionenaustauscher (d) ausschließlich der Aminkomponente zugesetzt.

Geeignete Radikalbildner für die Peroxidkomponente sind in erster Linie Diacylperoxide. Beispielhaft seien genannt Dibenzoylperoxid, Dilaurylperoxid und Bis(p-chlorbenzoyl-peroxid). Die Menge an Peroxid beträgt im Allgemeinen 0.05 bis 2 Gew.%, vorzugsweise 0,1 bis 0,8 Gew.-% bezogen auf die Peroxidkomponente.

Die Aminkomponente enthält aromatische Amine, wie z.B. N,N-Dimethyl-p-touidin, Bis-(2-hydroxyetyl)-p-toluidin, Bis-(N,N-2-hydroxyetyl)-3,5-dimetylanilin oder N-Metyl-N-(2-methylcarbamoyloxypropyl)-3,5-dimethylanilin. Die Menge an aromatischem Amin beträgt 0.1 bis 3 Gew.%, vorzugsweise 0,2 bis 1 Gew.-% bezogen auf die Aminkomponente.

Mit üblichen Zuschlagsstoffen (c) sind Stoffe und Substanzen gemeint, die in handelsüblichen Dentalmassen enthalten sind. Hierzu gehören Polymerisationsinhibitoren wie z.B. Hydrochinonmonometylether, Resorcin und 2,6-Di-tert.-butyl-4-methylphenol. Durch den Zusatz der Polymerisationsinhibitoren kann eine vorzeitige Polymerisation der Dentalmassen unterdrückt werden. Die Inhibitoren werden in Mengen von 50 bis 5000 ppm, vorzugsweise 100 bis 1000 ppm bezogen auf die Dentalmasse angewendet.

Als weitere Zuschlagsstoffe (c) können die erfindungsgemäßen Dentalmassen Füllstoffe in Form feiner Pulver mit einer mittleren Teilchengröße von 0,05 bis 100 µm, vorzugsweise von 0,1 bis 30µm enthalten. Die Füllstoffe können beispielsweise aus Siliziumdioxid, Quarz, Aluminiumoxid, Glas oder Glaskeramik bestehen. Der Anteil an Füllstoff kann bis zu 40 Gew.%, vorzugsweise bis zu 25 Gew.% bezogen auf die Dentalmasse betragen.

Selbstverständlich ist es möglich, übliche anorganische und organische Pigmente als Zuschlagsstoff (c) zuzusetzen, um der erfindungsgemäßen Dentalmasse ein zahnähnliches Aussehen zu geben.

Erfindungsgemäß geeignete Anionenaustauscher (d) sind vernetzte Polymere, die Aminogruppen, insbesondere Dimethylaminogruppen oder quatemäre Ammoniumgruppen als kovalent gebundene funktionelle Gruppen enthalten. Die Polymergrundlage des Anionenaustauschers ist vorzugsweise ein Styrol-Divinylbenzol-Copolymerisat. Weitere Einzelheiten zur Struktur, Herstellung und Eigenschaften von Ionenaustauschern werden beispielsweise in Kirk-Othmer, Encyclopedia of Chemical Technologie, Fourth Edition, Volume 14, S. 737 ff ausführlich beschrieben. Bevorzugt werden die sogenannten stark basischen Anionenaustauscher, d.h. Anionenaustauscher, die als funktionelle Gruppen Trimetylammoniumgruppen oder Hydroxyethyldimetylammoniumgruppen enthalten. Es sind sowohl gelförmige als auch makroporöse Anionenaustauscher einsetzbar. Die stark basischen Ionenaustauscher werden in der OH-Form eingesetzt.

Die Teilchengröße der für die vorliegende Erfindung eingesetzten Anionenaustauscher beträgt 0,1 bis 100 µm, vorzugsweise 0,5 bis 50 µm, besonders bevorzugt 1 bis 25 µm. Diese Teilchengrößen werden zweckmäßigerweise durch Mahlung gröberer, handelsüblicher Anionenaustauscher erzeugt. Als Mahlaggregate haben sich dabei Kugelmühlen und Perlmühlen besonders bewährt. Die Anwendung von flüssigem Stickstoff zur Abkühlung des Mahlgutes ist nicht zwingend erforderlich, sie erleichtert aber die Zerkleinerung, insbesondere dann, wenn besonders kleine Teilchengrößen angestrebt werden.

Anionenaustauscher werden üblicherweise als wasserhaltige Gele mit einem Wassergehalt von 35 bis 70% vertrieben. Für den erfindungsgemäßen Einsatz wird der Wassergehalt durch Trocknung auf weniger als 10%, bevorzugt weniger als 2% herabgesetzt. Die Trocknung kann beispielsweise in einem Trockenschrank bei Temperaturen von 60 bis 100°C und Drücken von 0,05 bis 1,0 bar erfolgen. Es ist sinnvoll, die Trocknung vor der Zerkleinerung vorzunehmen. Entsprechend betrifft die Erfindung auch ein Verfahren zur Herstellung härtbarer Dentalmassen, bei dem bei einem kommerziell erhältlichen Anionenaustauscher der Wassergehalt durch Trocknung auf weniger als 10% herabgesetzt wird, der Anionenaustauscher zerkleinert wird, und anschließend den Komponenten
a) radikalisch polymerisierbare Monomere,
b) Radikalbildner und/oder Cointiatoren,
c) übliche Zuschlagsstoffe,
als Komponente d) zugefügt wird.
Der Gewichtsanteil des Anionenaustauschers an der härtbaren Dentalmasse beträgt in der Regel 0,5 bis 50 %, vorzugsweise 4 bis 20 %.

Es ist überraschend, dass die feinteiligen Anionenaustauscher nach Aushärtung der Dentalmasse als integraler Bestandteil fest und dauerhaft in dem gebildeten Werkstoff verankert sind und keine Beeinträchtigung der mechanischen Eigenschaften des Werkstoffs beobachtet werden kann.

Die folgenden Beispiele dienen der Erläuterung der Erfindung, ohne sie zu beschränken.

### Beispiele:

Die erfindungsgemäßen Dentalmassen werden durch intensives Vermischen der in den folgenden Beispielen 1 bis 4 aufgeführten Bestandteile erzeugt.

### Beispiel 1

**BPO-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 0,3 | Benzoylperoxid |

**Amin-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 0,5 | Dimethyt-p-Toluidin |

### Beispiel 2

**BPO-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 0,3 | Benzoylperoxid |

**Amin-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 8 | stark basischer anionischer Ionenaustauscher in der OH-Form mit einem Wassergehalt von 0,2 Gew.-% und einer mittleren Teilchengröße von ca. 10 µm und eine maximalen Teilchengröße von ca. 100 µm. |
| 0,5 | Dimethyl-p-Toluidin |

### Beispiel 3

**BPO-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 0,3 | Benzoylperoxid |

**Amin-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 16 | anionischer Ionenaustauscher wie in Beispiel 2 beschrieben |
| 0,5 | Dimethyl-p-toluidin |

### Beispiel 4

**BPO-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 0,3 | Benzoylperoxid |

**Amin-Komponente (in Gew.-Teilen)**

| | |
|---|---|
| 80 | Urethandimethacrylat nach Formel 3 |
| 10 | 2-Hydroxyethylmethacrylat |
| 10 | Triethylenglycoldimethacrylat |
| 32 | anionischer Ionenaustauscher wie in Beispiel 2 beschrieben |
| 0,5 | Dimethyl-p-toluidin |

### Beispiel 5 (Anwendungstest, Bindungsfestigkeit an Schmelz und Dentin)

Die Wirksamkeit und Eignung der Dentalmassen als Bondingkomponenten werden in Kombination mit einem selbst-ätzenden Primer Adhäsiv durch Bestimmung der Scherbindungsfestigkeit auf Schmelz und Dentin überprüft. Extrahierte, menschliche Mahlzähne, die für maximal sechs Monate nach der Entfernung in 1 % Chloraminlösung aufbewahrt waren, werden nach sorgfältiger Reinigung unter entionisiertem Wasser mit Druckluft getrocknet und in zylindrischen Gummiformen (Durchmesser: 25 mm; Höhe; 15 mm) mit Epoxidharz (Lekutherm X20, Härter T1) so eingebettet, dass eine unversehrte Proximalfläche auf dem Boden der Gummiform aufliegt. Nach 24 Stunden Härtungszeit bei Raumtemperatur (23° C) werden die eingebetteten Proben aus den Formen befreit und von der Bodenseite her auf SiC-Papier der Kömungen 180, 240, 320, 400 und schließlich 600 nass abgeschliffen, bis eine ausreichend große, periphere Schmelz- oder Dentinoberfläche zur Anbindung eines Kompositzylinders mit 3,5 mm Durchmesser freiliegt.

Auf die so freigelegten Zahnoberflächen wird nach Abwaschen mit entionisiertem Wasser und Trocknung im Druckluftstrom das handelsübliche selbst-ätzende Primer Adhäsiv **iBond** *Gluma inside* (pH 2,2) von Heraeus Kulzer GmbH & Co. KG mit einem gesättigten Pinsel in drei unmittelbar aufeinander folgenden Schichten aufgetragen und für 30 Sekunden ungestört auf der behandelten Fläche belassen. Anschließend wird die Schicht 5 bis 10 Sekunden lang im milden Druckluftstrom sorgfältig vom Lösungsmittel (Aceton und Wasser) befreit. In einer ersten Versuchsreihe wird das Adhäsiv nicht lichtaktiviert. In einer zweiten Versuchsreihe wird iBond für 30 Sekunden mit der Polymerisationsleuchte Translux CL (Heraeus Kulzer; 600 mW/cm² Leistung) aktiviert.

Die vorbehandelten Proben werden in einer Einspannvorrichtung unter einer teilbaren Teflonform (Durchmesser 3,5 mm, Höhe 2,0 mm) eingespannt. Als redox-aktviertes Komposit wird das Stumpfaufbaumaterial Core Paste (Den-Mat, Santa Maria, CA, USA) nach Herstellerangabe aus gleichen Volumenteilen Basis- und Katalysatorpaste angemischt, in eine Applikationsspritze aufgenommen, in die Teflonform eingebracht, mit einem O₂-undurchlässigen Strip abgedeckt und 15 Minuten lang bei Raumtemperatur belassen. Als Referenzproben dienen mit iBond vorbehandelte Zahnflächen, auf denen das Material Core Paste ohne Zwischenschicht direkt aufgebracht wird. Anschließend werden die Proben aus der Form befreit und für 24 Stunden in 37° C warmem Wasser gelagert.

Zur Prüfung der erfindungsgemäßen Formulierungen (Beispiele 1 bis 4) werden jeweils die BPO- und die aminhaltige Komponente zu gleichen Volumenanteilen vermischt und mit einem gesättigten Pinsel auf die mit iBond vorbehandelte Zahnfläche in einer Schicht aufgetragen und 60 Sekunden lang ungestört belassen, bevor die Monomerschicht im sanften Luftstrom zu einer gleichmäßig dünnen Schicht verblasen wird. Auf die so vorbehandelten Proben wird nach Einspannen unter einer Teflonform das Stumpfaufbaumaterial Core Paste, wie oben beschrieben, appliziert und für 24 Stunden in 37° C Wasser gelagert.

Die Proben werden in einer Universalprüfmaschine eingespannt und mit Hilfe eines Druckstempels auf dem Kunststoffzylinder parallel zu und in einem Abstand von ca. 0,2 mm von der Zahnoberfläche bei einer Vorschubgeschwindigkeit von 1 mm/Minute bis zum Bruch belastet.

Die Scherbindungsfestigkeit errechnet sich aus dem Quotienten aus Bruchkraft und Anbindungsfläche des Zylinders und wird in MPa angegeben.

An jeder abgescherten Probe wird auf der Zahnseite die Frakturmorphologie im Stereomikroskop bei 20-facher Vergrößerung inspiziert und als kohäsives Versagen im Zahn oder Kunststoff, als adhäsives Versagen an der Grenzfläche oder als gemischte Fraktur (adhäsiv-kohäsiv) klassifiziert.

In zusätzlichen Versuchen wird der Einfluss verkürzter Verweilzeiten (10 oder 30 Sekunden) der erfindungsgemäßen Formulierungen auf die Bindungsfestigkeit geprüft.

Die Ergebnisse sind in den folgenden Tabellen 1 bis 12 zusammengestellt:

**Tabelle 1 Scherbindungsfestigkeit auf Dentin (Keine Lichtaktivierung von iBond)**

| Zubereitung nach Beispiel Nr. | Scherbindungsfestigkeit (MPa) | | Signif. Unterschiede (p<0.05) |
|---|---|---|---|
| | Mittelwert (MPa) n = 6 | Standardabweichung ± (σₙ₋₁) | |
| Referenz | 0,0 | - | a |
| 1 | 8,5 | 1,5 | b |
| 2 | 19,9 | 4,1 | c |
| 3 | 19,7 | 7,4 | c |
| 4 | 22,8 | 8,0 | c |

| | | | |
|---|---|---|---|
| Varianzanalyse: p < 0,0001. Werte mit gleichen Buchstaben sind nicht signifikant unterschiedlich (Duncan Ranking: p<0,05). | | | |

Mit keiner der Referenzproben ohne Bond-Zwischenschicht wird eine Anbindung an Dentin erzielt. Alle Proben lösen sich spontan beim Entfernen aus der Teflonform. Die nicht mit lonenaustauscher versehene Zubereitung 1 zeigt eine signifikant geringere Bindungsfestigkeit als die mit den Zubereitungen 2 bis 4 beschichteten Proben, während statistisch kein Unterschied in der Wirksamkeit der Zubereitungen mit unterschiedlicher Ionenaustauscher-Konzentration nachgewiesen wird. Das Versagen der Referenzproben lässt sich wahrscheinlich teilweise mit der die Aminkomponente des Komposits desaktivierenden Wirkung der sauren Monomerbestandteile von iBond erklären. Gleichzeitig darf man davon ausgehen, dass aufgrund von O₂₋Inhibition der iBond Polymerisation, die durch Diffusion von Sauerstoff sowohl an der freien als auch kontinuierlich an der Dentinseite erfolgt, und aufgrund der langsamen Redoxpolymerisation des Komposits eine Härtung an der Grenzfläche verhindert wird. Sofern eine Schicht der Zubereitung 1, die keinen Ionenaustauscher enthält, aufgetragen wird, findet hingegen eine Polymerisation an der Grenzfläche zum Dentin statt. Zubereitung 1 bindet innerhalb von drei bis vier Minuten nach dem Zusammenmischen der Komponenten ab, d.h. die Diffusionszeit für das saure Monomer ist relativ kurz und eine vollständige Desaktivierung der Aminkomponente der experimentellen Zubereitung wird verhindert. Zubereitungen, die Ionenaustauscher in den getesteten Konzentrationen von 8, 16 oder 32% in der Aminlösung enthalten, sind gleich gut wirksame Komponenten zur Erzielung hoher Bindungsfestigkeit am Dentin. Die Wirksamkeit lässt sich nur über die erfindungsgemäße Schutzfunktion der Aminkomponente durch den basischen Ionenaustauscher in den redoxaktivierten Zubereitungen erklären, da die Abbindezeit der Abmischungen nicht unterschiedlich von der der Zubereitung 1 ist.

**Tabelle 2 Scherbindungsfestigkeit auf Dentin (30 Sekunden Lichtaktivierung von iBond)**

| Zubereitung nach Beispiel Nr. | Scherbindungsfestigkeit (MPa) Signif. | | Unterschiede (p<0.05) |
|---|---|---|---|
| | Mittelwert (MPa) n = 6 | Standardabweichung ± (σₙ₋₁) | |
| Referenz | 0,0 | - | a |
| 1 | 5,2 | 4,6 | b |
| 2 | 13,8 | 3,4 | c |
| 3 | 17,7 | 5,1 | c |
| 4 | 15,8 | 1,4 | c |

| | | | |
|---|---|---|---|
| Varianzanalyse: p < 0,0001. Werte mit gleichen Buchstaben sind nicht signifikant unterschiedlich (Duncan Ranking: p<0,05). | | | |

Lichtaktivierung von iBond führt für die Zubereitungen nach Beispiel 1 bis 4 zu niedrigerer Bindungsfestigkeit am Dentin als nicht aktivierte iBond-Schichten. Die Wirksamkeit der Ionenaustauscher enthaltenden Proben ist jedoch auch nach diesem Vorgehen signifikant besser als die der unbeladenen Abmischung 1 und der Referenz.

**Tabelle 3 Scherbindungsfestigkeit auf Dentin abhängig von Verweilzeit der Zubereitung nach Beispiel 2 (Keine Lichtaktivierung von iBond)**

| Verweilzeit (Sekunden) | Scherbindungsfestigkeit (MPa) | | Signif. Unterschiede p<0.05 |
|---|---|---|---|
| | Mittelwert (MPa) n = 6 | Standardabweichung ± (σₙ₋₁) | |
| 10 | 17,0 | 3,5 | a |
| 30 | 21,2 | 5,0 | a |
| 60 | 19,9 | 4,1 | a |

| | | | |
|---|---|---|---|
| Varianzanalyse: p = 0,2462. Werte mit gleichen Buchstaben sind nicht signifikant unterschiedlich (Duncan Ranking: p<0,05). | | | |

Die getesteten, kürzeren Verweilzeiten (10 und 30 Sekunden) der Zubereitung nach Beispiel 2 auf mit iBond behandeltem Dentin sind nicht signifikant unterschiedlich von einer Verweilzeit von einer Minute.

**Tabelle 4 Scherbindungsfestigkeit auf Dentin abhängig von Verweilzeit der Zubereitung nach Beispiel 2 (30 Sekunden Lichtaktivierung von iBond)**

| Verweilzeit (Sekunden) | Scherbindungsfestigkeit (MPa) | | Signif. Unterschiede (p<0.05) |
|---|---|---|---|
| | Mittelwert (MPa) n = 6 | Standardabweichung ± (σₙ₋₁) | |
| 10 | 14,2 | 3,0 | a |
| 30 | 17,7 | 4,4 | a |
| 60 | 13,8 | 3,4 | a |

| | | | |
|---|---|---|---|
| Varianzanalyse: p = 0,1725. Werte mit gleichen Buchstaben sind nicht signifikant unterschiedlich (Duncan Ranking: p > 0,05). | | | |

Die getesteten, kürzeren Verweilzeiten (10 und 30 Sekunden) der Zubereitung nach Beispiel 2 auf mit iBond behandeltem und lichtaktiviertem Dentin sind nicht signifikant unterschiedlich von einer Verweilzeit von einer Minute. Die mittlere Bindungsfestigkeit ist hingegen geringer als in der Gruppe ohne Lichtaktivierung von iBond (Tabelle 3).

**Tabelle 5 Scherbindungsfestigkeit auf Schmelz (Keine Lichtaktivierung von iBond)**

| Zubereitung nach Beispiel Nr. | Scherbindungsfestigkeit (MPa) | | Signif. Unterschiede (p<0.05) |
|---|---|---|---|
| | Mittelwert (MPa) n = 6 | Standardabweichung ± (σₙ₋₁) | |
| Referenz | 21,5 | 6,5 | a |
| 1 | 31,8 | 5,5 | b |
| 2 | 27,4 | 5,3 | a, b |
| 3 | 25,7 | 3,3 | a, b |
| 4 | 28,8 | 2,5 | b |

| | | | |
|---|---|---|---|
| varianzanalyse: p = 0,017. Werte mit gleichen Buchstaben sind nicht signifikant unterschiedlich (Duncan Ranking: p<0,05). | | | |

Die Referenzproben ohne Bond-Zwischenschichten zeigen eine geringere Bindungsfestigkeit als die mit den Zubereitungen 1 bis 4 beschichteten Proben. Hypothetisch lässt sich die erstaunlich hohe Bindungsfestigkeit der Referenzproben damit erklären, dass die Dicke der iBond-Schicht wegen der sehr geringen Ätztiefe am Schmelz, aufgrund des gründlichen Verblasens nach Applikation und aufgrund von Verdrängung durch die vergleichsweise hohe Viskosität der Kompositpaste nur außerordentlich gering ist. Das bei Diffusion die Aminkomponente des Komposits angreifende, sehr geringe Volumen an saurem Monomer aus iBond ist vermutlich zu gering, um eine Polymerisation entlang der Grenzfläche zu verhindern. Gleichzeitig trägt vermutlich die Diffusion der Kompositmonomere in die unpolymerisierte (lichtaktivierbare) iBond-Schicht dazu bei, dass aufgrund von Radikaltransfer der Haftvermittler polymerisiert. Die Zubereitungen 1 bis 4 sind nach der statistischen Analyse als gleich effektiv anzusehen. Die Bindungsfestigkeiten geben keinen Hinweis darauf, dass Zusatz des Ionenaustauschers in den gewählten Konzentrationen einen Einfluss auf die Wirksamkeit am Schmelz hat.

**Tabelle 6 Scherbindungsfestigkeit auf Schmelz (30 Sekunden Lichtaktivierung von iBond)**

| Zubereitung nach Beispiel Nr. | Scherbindungsfestigkeit (MPa) | | Signif. Unterschiede (p<0.05) |
|---|---|---|---|
| | Mittelwert (MPa) n = 6 | Standardabweichung ± (σₙ₋₁) | |
| Referenz | 25,0 | 4,7 | a |
| 1 | 27,6 | 4,1 | a |
| 2 | 28,7 | 3,9 | a |
| 3 | 29,5 | 3,5 | a |
| 4 | 27,1 | 5,1 | a |

| | | | |
|---|---|---|---|
| Varianzanalyse: p = 0,017. Werte mit gleichen Buchstaben sind nicht signifikant unterschiedlich (Duncan Ranking: p<0,05). | | | |

Alle Proben, einschließlich der Referenz weisen vergleichbar hohe Bindungsfestigkeit am Schmelz auf. Die etwas höhere Festigkeit der Referenz im Vergleich mit der nicht vorbelichteten Gruppe ist vermutlich auf die Vorpolymerisation von iBond zurückzuführen, nach der nur noch das nunmehr sehr geringere Volumen der iBond Inhibitionsschicht als Säurelieferant und damit als das Amin der Redoxkomponenten kompromittierende Agens zur Verfügung steht.

**Tabelle 7 Scherbindungsfestigkeit auf Schmelz abhängig von Verweilzeit der Zubereitung nach Beispiel 2 (Keine Lichtaktivierung von iBond)**

| Verweilzeit (Sekunden) | Scherbindungsfestigkeit (MPa) | | Signif. Unterschiede (p<0.05) |
|---|---|---|---|
| | Mittelwert (MPa) n = 6 | Standardabweichung ± (σₙ₋₁) | |
| 10 | 29,4 | 4,2 | b |
| 30 | 22,2 | 5,8 | a |
| 60 | 27,4 | 5,3 | a, b |

| | | | |
|---|---|---|---|
| Varianzanalyse: p < 0,0680. Werte mit gleichen Buchstaben sind nicht signifikant unterschiedlich (Duncan Ranking: p<0,05). | | | |

Die getesteten Verweilzeiten der Zubereitung 2 zeigen keinen Unterschied in der Bindungsfestigkeit am Schmelz. Zehn Sekunden werden daher als ausreichend erachtet.

**Tabelle 8 Scherbindungsfestigkeit auf Schmelz abhängig von Verweilzeit der Zubereitung nach Beispiel 2 (30 Sekunden Lichtaktivierung von iBond)**

| Verweilzeit (Sekunden) | Scherbindungsfestigkeit (MPa) | | Signif. Unterschiede (p<0-05) |
|---|---|---|---|
| | Mittelwert (MPa) n = 6 | Standardabweichung ± (σₙ₋₁) | |
| 10 | 26,0 | 3,5 | a |
| 30 | 26,8 | 4,4 | a |
| 60 | 28,7 | 3,9 | a |

| | | | |
|---|---|---|---|
| Varianzanalyse: p < 0,4958. Werte mit gleichen Buchstaben sind nicht signifikant unterschiedlich (Duncan Ranking: p<0,05). | | | |

Die getesteten Verweilzeiten der Zubereitung 2 zeigen auch nach Lichtaktivierung von iBond keinen Unterschied in der Bindungsfestigkeit am Schmelz.

Bei der stereomikroskopischen Beurteilung der abgescherten Proben (Tabellen 9 bis 12) zeigen die Referenzproben an Dentin ausschließlich adhäsives Versagen. Fast alle anderen Proben, sowohl an Schmelz als auch an Dentin, weisen kohäsive Frakturen auf. Von den 48 Dentinproben, die mit den Zubereitungen 1 bis 4 versehen waren, liegt in 42 Fällen die Fraktur im Resin, in zwei Fällen im Dentin und in vier Fällen an der Grenzfläche. Von den insgesamt 60 Schmelzproben weisen 24 Fälle Resinfrakturen und 36 Schmelzfrakturen auf. Kohäsiv-adhäsive Mischfrakturen wurden nicht angetroffen. Das Auftreten von kohäsivem Versagen in der Zahnhartsubstanz oder im aufpolymerisierten Resin macht deutlich, dass die Bindung an der Grenzfläche stärker ist als die des darunter- oder darüberliegenden Substrats.

**Tabelle 9 Frakturmorphologie (Dentin) (Keine Lichtaktivierung von iBond)**

| Zubereitung nach Beispiel Nr. | Zahn (kohäsiv) | Resin (kohäsiv) | Grenzfläche (adhäsiv) |
|---|---|---|---|
| Referenz | 0 | 0 | 6 |
| 1 | 0 | 4 | 2 |
| 2 | 0 | 5 | 1 |
| 3 | 0 | 5 | 1 |
| 4 | 1 | 5 | 0 |

**Tabelle 10 Frakturmorphologie (Dentin) (30 Sekunden Lichtaktivierung von iBond)**

| Zubereitung nach Beispiel Nr. | Zahn (kohäsiv) | Resin (kohäsiv) | Grenzfläche (adhäsiv) |
|---|---|---|---|
| Referenz | 0 | 0 | 6 |
| 1 | 0 | 6 | 0 |
| 2 | 1 | 5 | 0 |
| 3 | 0 | 6 | 0 |
| 4 | 0 | 6 | 0 |

**Tabelle 11 Frakturmorphologie (Schmelz) (Keine Lichtaktivierung vnn iBond)**

| Zubereitung nach Beispiel Nr. | Zahn (kohäsiv) | Resin (kohäsiv) | Grenzfläche (adhäsiv) |
|---|---|---|---|
| Referenz | 2 | 4 | 0 |
| 1 | 5 | 1 | 0 |
| 2 | 5 | 1 | 0 |
| 3 | 4 | 2 | 0 |
| 4 | 3 | 3 | 0 |

**Tabelle 12 Frakturmorphologie (Schmelz) (30 Sekunden Lichtaktivierung von iBond)**

| Zubereitung nach Beispiel Nr. | Zahn (kohäsiv) | Resin (kohäsiv) | Grenzfläche (adhäsiv) |
|---|---|---|---|
| Referenz | 2 | 4 | 0 |
| 1 | 4 | 2 | 0 |
| 2 | 4 | 2 | 0 |
| 3 | 4 | 2 | 0 |
| 4 | 3 | 3 | 0 |

## Patentansprüche

1. Härtbare Dentalmassen aus einem Zweikomponentensystemen aus Peroxid- und Aminkomponenten zur Härtung auf mit sauren Komponenten behandelten Zahnflächen, wobei die Aminkomponenten
a) radikalisch polymerisierbare Monomere,
b) Radikalbildner und/oder Cointiatoren,
c) übliche Zuschlagsstoffe und
d) feinteiligen stark basischen Anionenaustauscher in der OH-Form enthalten.

2. Härtbare Dentalmasse nach Anspruch 1, **dadurch gekennzeichnet, dass** der GewichtsAnteil des Anionenaustauschers 0,5 bis 50 % bezogen auf die Dentalmasse beträgt.

3. Verfahren zur Herstellung der Aminkomponenten härtbarer Dentalmassen, die aus einem Zweikomponentensystemen aus Peroxid- und Aminkomponenten bestehen, **dadurch gekennzeichnet, dass**
■ bei einem kommerziell erhältlichen stark basischen Anionenaustauscher in der OH-Form der Wassergehalt durch Trocknung auf weniger als 10% herabgesetzt wird,
■ der Anionenaustauscher zerkleinert wird,
■ und anschließend den Komponenten a), b) und c) gemäß Anspruch 1 zugefügt wird.

4. Verfahren nach Anspruch 3, **dadurch gekennzeichnet, dass** der Wassergehalt auf weniger als 2% herabgesetzt wird.

5. Verwendung von Aminkomponenten härtbarer Dentalmassen mit den Komponenten a). b), c), d) gemäß Anspruch 1 zur Herstellung von Dentalfüllungsmaterialien, Haftvermittlern, adhäsiven Zahnlacken, Versieglern oder Befestigungsmaterialien.

## Claims

1. Curable dental material consisting of a two-component system of peroxide and amine components for curing on dental surfaces treated with acidic components, the amine components containing
a) radically polymerisable monomers,
b) radical formers and/or co-initiators
c) common additives and
d) finely particulate, highly basic anion exchangr in the OH form.

2. Curable dental compounds according to claim 1 **characterised in that** the proportion by weight of the anion exchanger is 0.5 to 50 %, based on the total mass of the dental material.

3. Process for the production of the amine components of curable dental materials which consist of a two-component system of peroxide and amine components, **characterised in that**
• the water content of a commercially available highly basic anion exchanger in the OH form, is reduced to less than 10% by drying
• the anion exchanger is comminuted,
• and subsequently added to the components a), b) and c) according to claim 1.

4. Process according to claim 3 **characterised in that** the water content is reduced to less than 2%.

5. Use of amine components of curable dental materials with the components a), b), c), d) according to claim 1 for the production of dental filling materials, coupling agents, adhesive tooth varnishes, sealants or fixing materials.

## Revendications

1. Masses dentaires durcissables composées d'un système bicomposant en composants de peroxyde et d'amine en vue du durcissement sur des surfaces dentaires traitées avec des composants acides, les composants d'amine contenant
a) des monomères polymérisables par voie radicalaire,
b) des formateurs de radicaux et/ou des co-amorceurs,
c) des agrégats habituels et
d) des échangeurs d'anions finement dispersés fortement basiques sous la forme OH.

2. Masse dentaire durcissable selon la revendication 1, **caractérisée en ce que** la proportion pondérale de l'échangeur d'anions est comprise entre 0,5 et 50 % par rapport à la masse dentaire.

3. Procédé en vue de la fabrication des composants d'amine de masses dentaires durcissables, qui se composent d'un système bicomposant en composants de peroxyde et d'amine, **caractérisé en ce que**
• la teneur en eau d' un échangeur d'anions fortement basique sous la forme OH disponible dans le commerce est réduite à moins de 10 % par séchage,
• l'échangeur d'anions est fractionné,
• et est ensuite ajouté aux composants a), b) et c) selon la revendication 1.

4. Procédé selon la revendication 3, **caractérisé en ce que** la teneur en eau est réduite à moins de 2 %.

5. Utilisation de composants d'amine de masses dentaires durcissables avec les composants a), b), c), d) selon la revendication 1 en vue de la fabrication de matériaux de remplissage dentaire, de promoteurs d'adhésion, de vernis dentaires adhésifs, d'agents de scellement ou de matériaux de fixation.
